# EUROPEAN PATENT APPLICATION

(11) **EP 1 886 618 A1**
(43) Date of publication of application: **13.02.2008**
(21) Application number: 06746400.8
(22) Date of filing: 16.05.2006
(51) Int. Cl.: A61B 1/00, A61B 5/07

(54) **CAPSULE ENDOSCOPE**

(30) Priority: 03.06.2005 JP 2005163689
(71) Applicant: Konica Minolta Medical & Graphic, Inc., Tokyo 163-0512 (JP)
(72) Inventor: HABU, Takeshi Konica Minolta Medical & Graphic Inc, Shinjuku-ku, Tokyo 1630512 (JP); SAKUMA, Haruhiko Konica Minolta Medical&GraphicInc, Shinjuku-ku, Tokyo 1630512 (JP); TAKEYAMA, Toshihisa KonicaMinolta Med.&Graphic Inc, Shinjuku-ku, Tokyo 1630512 (JP); GOTO, Narito Konica Minolta Medical & Graphic, Inc, Shinjuku-ku, Tokyo 1630512 (JP); UMEKI, Mamoru Konica Minolta Medical&Graphic, Inc., Shinjuku-ku, Tokyo 1630512 (JP)
(74) Representative: Henkel, Feiler & Hänzel
(86) International application number: PCT/JP2006/309686
(87) International publication number: WO 2006/129472

(57) **Abstract**

It is a feature in the present invention to provide a capsule endoscope, specifically in the form of tablet capsule integrated with an objective lens, an illuminating source section, a solid-state image sensor and so forth, and also to provide a medical capsule endoscope exhibiting excellent durability as well as sharp images, accompanied with smooth movement in any of digestive organs in body cavities. Also disclosed is a medical capsule endoscope of the present invention comprising surface area A having a wettability index of at least 40 mN/m.

## Description

### TECHNICAL FIELD

The present invention relates to a capsule endoscope, specifically in the form of tablet capsule integrated with an objective lens, an illuminating source section, a solid-state image sensor and so forth, and to a medical capsule endoscope exhibiting sharp images, accompanied with smooth movement in any of digestive organs in body cavities.

### BACKGROUND

Presently, in the field of medicine, a fiber scope and an electronic endoscopic apparatus have widely been utilized for diagnosis and treatment in the inside of body cavity. Each of these apparatuses comprises a tubular insertion section equipped with an image sensor or such at the end, an operation section connected to this insertion section, an image processor connected thereto, a display apparatus and so forth, and is an endoscopic apparatus to inspect and observe desired portions in the inside of body cavity by inserting the insertion section into the inside of body cavity of an object to sense images. In such the endoscopic apparatus, sufficiently skilled doctors and engineers should have operated it because of a large size and length of the insertion section inserted in the inside of body cavity, and its complicated shape to conduct inspection and observation. These apparatuses also cause pain to the object during insertion to the inside of body cavity.

In view of the above-described situation, recently developed has been an ultrasmall endoscope, which is a so-called capsule endoscope, fitted with a solid-state image sensor having a photographic optics system in the inside of a tablet-capsule-shaped enclosure and so forth. This capsule endoscope swallowed by the object is easily inserted into the inside of body cavity. By this, organs such as a small intestine and so forth have easily been observed and inspected employing a wireless communication device by which images of an affected area are taken, the image data are transmitted from the inside of a body, and the signal is received outside the body (refer to Patent Document 1, for example).
Patent Document 1: Japanese Patent O.P.I. Publication No. 7-289504.

### DISCLOSURE OF THE INVENTION

### PROBLEMS TO BE SOLVED BY THE INVENTION

However, designing suitable for the internal surface of digestive tract in which digestive fluid is secreted is desired in order to take a picture of the inside of the body cavity smoothly, the situation still remains at a point focused only on a property exhibiting excellent hydrophilicity. The surface situation where a surface member of a capsule endoscope is strongly acidic like pH 1 - 2 when passing through a gastric region, and is oppositely neutral or mildly alkaline in the small and large intestines. Decline in transmittance and light emission quantity is caused since the lens surface and the lighting apparatus surface are coated by the digestive fluid secreted from the digestive tract, resulting in unsharp image-sensing images. The endoscope can not be smoothly moved in digestive organs though depending on adherence of the digestive fluid or secretion, and a convexoconcave situation on the internal surface of digestive organs, whereby decline in unsharpness of image-sensing images is presently generated.

The present invention was made on the basis of the above-described situation. It is an object of the present invention to provide a capsule endoscope, specifically in the form of tablet capsule integrated with an objective lens, an illuminating source section, a solid-state image sensor and so forth, and also a medical capsule endoscope exhibiting excellent durability as well as sharp images, accompanied with smooth movement in any of digestive organs in body cavities.

### MEANS TO SOLVE THE PROBLEMS

The above-described object can be accomplished by the following structures.
(Structure 1) A capsule endoscope comprising surface area A having a wettability index of at least 40 mN/m.
(Structure 2) The capsule endoscope of Structure 1, wherein the surface area A is formed from a protective member of a photographing section comprising a lens and an illumination unit.
(Structure 3) The capsule endoscope of Structure 1, wherein the surface area A is a lens surface or an illumination unit surface.
(Structure 4) The capsule endoscope of any one of Structures 1 - 3, wherein the surface area A is corona-discharge-treated or plasma-discharge-treated.
(Structure 5) The capsule endoscope of any one of Structures 1 - 4, wherein a material constituting the surface area A is a polymer having a nonionic group or an anionic group.
(Structure 6) The capsule endoscope of Structure 5, wherein the nonionic group comprises a sugar chain structure having a single hydroxyl group or a plurality of hydroxyl groups, or an alkylene oxide group.
(Structure 7) The capsule endoscope of Structure 5, wherein the anionic group is a sulfonic acid group or a phosphoric acid group.

### EFFECT OF THE INVENTION

In the present invention, provided can be a capsule endoscope, specifically in the form of tablet capsule integrated with an objective lens, an illuminating source section, a solid-state image sensor and so forth, and also to provide a medical capsule endoscope exhibiting excellent durability as well as sharp images, accompanied with smooth movement in any of digestive organs in body cavities.

### BRIEF DESCRIPTION OF THE DRAWINGS

Fig. 1 is an overall schematic diagram showing an example of a capsule endoscope in the embodiment of the present invention.
Fig. 2 is an overall schematic diagram showing another example of a capsule endoscope in the embodiment of the present invention.

### EXPLANATION OF NUMERALS

- 1: Polymer enclosure of capsule endoscope
- 2: Objective lens
- 3: Image sensor
- 4: Light emission module
- 5: Light source section
- 6: Transmitting device
- 7: Receiving device
- 8: Antenna
- 9: Electric storage device
- 10: Chemical-keeping device
- S: Protective member of photographing section
- a: Long axis length
- b: Short axis length

### DESCRIPTION OF THE PREFERRED EMBODIMENTS

Next, the preferred embodiment of the present invention will be described in detail.

After considerable effort during intensive studies, the inventors have found out that a medical capsule endoscope exhibiting sharp images, accompanied with smooth movement in any of digestive organs in body cavities can be realized by using a capsule endoscope in the form of tablet capsule integrated with an objective lens, an illuminating source section, a solid-state image sensor and so forth, together with surface area A having at least a wettability index of 40 mN/m.

First, the overall structure of a capsule endoscope of the present invention will be described.

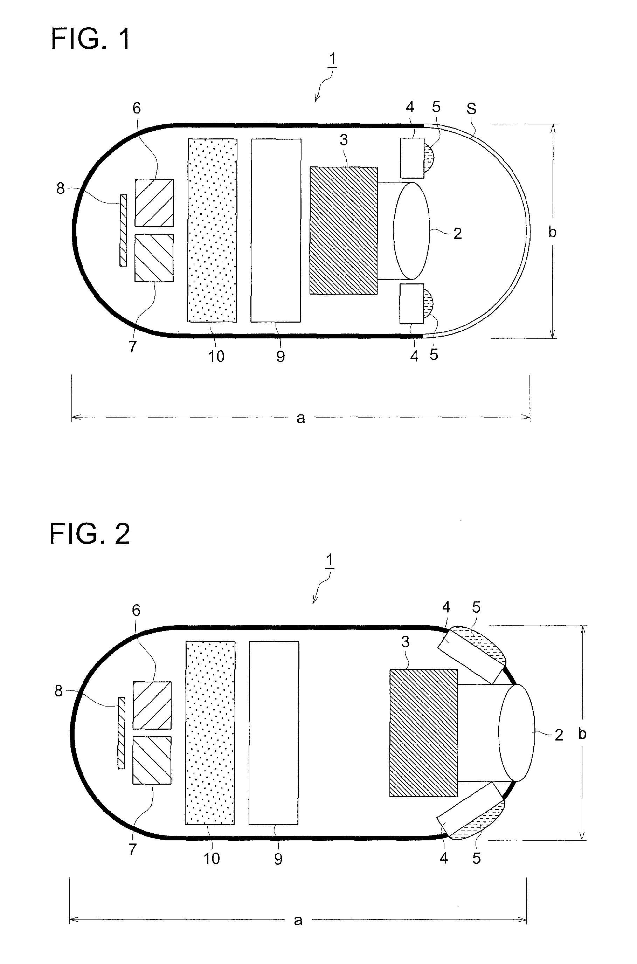

Fig. 1 is an overall schematic diagram showing an example of a capsule endoscope in the embodiment of the present invention.

As shown in Fig. 1, capsule endoscope 1 of the present invention is a so-called ultrasmall endoscope which is independently moved by being separated from an operating device capable of wireless transmission and reception, and is an electronic endoscope capable of wireless transmission and reception of photographed images. As an observing function device, the observation optical system possesses objective lens 2 and image sensor 3, and image sensor 3 employs fine CCD or CMOS having pixels placed at an interval of 0.1 - 3 µm. Further, light emission module 4 and light source section 5 constituting an illumination optical system conventionally utilize a white, green or red LED (hereinafter, referred to also as LED). Protective member of a photographing section S is provided outside these observation optical system and illumination optical system. In a capsule endoscope of the present invention, it is preferred that the surface of protective member S is arranged to be surface area A having a wettability index of at least 40 mN/m.

Wireless transmitting and receiving antenna 8 is placed with transmitting device 6, receiving device 7 and electric storage device 10 provided on the opposite side of the illumination optical system. Space area 9 is a room of storing a medical solution to be fed to the affected area, or is, in some situations, a room of storing the specimen after collecting a test-specimen in the affected area. Electric storage device 10 may be a transformer converting into an electric source to produce light emission of LED for illumination by an ultrasonic wave, magnetic field or electric field transmitted from the outside.

The capsule endoscope of the present invention should include at least an observation optical system, an illumination optical system, an antenna system, a transmitting and receiving system, and others are an electric storage system to supply energy, a DDS system, a test-specimen collection system and so forth.

The capsule endoscope is in the form of a small chicken egg so as to move smoothly in the upper and lower digestive organs, and the rough elliptical shape is preferable as a top view as well as a side view, but the spherical shape or rugby ball shape may be allowed to be used. In the case of the spherical shape, since the more spherical the shape, the more difficult the position control, photographing in a given direction should be adjusted. Concerning a capsule endoscope of the present invention, for example, photographing at desired portions becomes possible by employing the optical system described in Japanese Patent O.P.I. Publication No. 2001-174713. That is, since the total length of the optical system can be shortened in combination with reflecting surfaces, or reflecting and refracting surfaces according to the objective optical system, a small objective optical system capable of a wide angle observation in a small reflection space is realized. Further, a small endoscope capable of observation throughout the entire circumference in the circumference direction perpendicular to the axis direction of an endoscope can be obtained by applying the above-described objective optical system to the endoscope of the present invention.

When a cylindrical column circumscribing a capsule endoscope is first drawn, and the volume of the cylindrical column is set so as to be at a minimum, the a/b ratio is defined as an aspect ratio, provided that the diameter is short axis length b of the capsule endoscope, and the length of the cylindrical column is long axis length a of the capsule endoscope. In this case, an aspect ratio of at least 1 and at most 2.3 is preferable, and an aspect ratio of at least 1 and at most 2 is more preferable.

Concerning objective lens 2 to focus an observed image onto CCD or CMOS as image sensor 3, a single lens or a plurality of aspheric objective lenses, for example, is/are provided. In the case of employing these aspheric lenses, since a number of lenses can be reduced, the total length of a capsule section can be shortened, whereby a swallowable property can be improved.

As to the capsule endoscope of the present invention, a hydrophilic medium as a lubricative material is coated or surface-treated on the outer surface enclosing the capsule main body. In this case, when swallowing capsule endoscope 1, affinity of secretion in digestive organs for the hydrophilic medium is produced, and lubricity between foregoing capsule endoscope 1 and lumen of the gut is improved, whereby the endoscope can be smoothly moved with no damage of the gut to obtain sharp images.

Capsule endoscope 1 of the present invention includes an endoscope observation system fitted with an output signal processing system to drive image sensor 3 provided outside body cavities, an electric installation device to supply electric power to illumination device 4, and a liquid crystal display element in combination to sharply display endoscope images photographed by foregoing image sensor 3 on a liquid crystal monitor placed outside the body.

For example, the image processing via recording of photographed recording data obtained by a capsule endoscope on a small size recording medium is conducted to detect a diseased portion, whereby early detection becomes possible. The electric installation device is driven by an electric source for hospital use, an electric source for household use, a solar battery, a fuel battery or such.

Fig. 2 is an overall schematic diagram showing another example of a capsule endoscope in the embodiment of the present invention.

Similarly to Fig. 1 as described above, the basic structure is a structure constituting a part of the outermost portion as objective lens 2 and light source section 5 without providing protective member S outside the observation and illumination optical systems in Fig. 2, and it is preferred that the surface of objective lens 2 or light source section 5 is arranged to be surface area A having a wettability index of at least 40 mN/m.

Next, surface area A having a wettability index of at least 40 mN/m in the present invention will be described in detail.

As described before, the gastric portion digestive organs exhibit pH close to 1 as the condition of decomposing protein, but hydrophilicity under the condition of acidity is weak since a proton is provided to a carboxyl group in the case of a polymer having a carboxyl group as a hydrophilic group. On the other hand, it is preferable that hydrophilicity can be maintained even under the condition of strong acidity in the case of a polymer having a functional group such as an alkylene oxide group, a hydroxy group or such, which is a group ionically dissociated at pH being 1 like a sulfonic acid group or a hydrophilic group with no ionic dissociation. However, when such the polymer is employed, since water and salt are penetrated into the internal image sensing electric installation system through the polymer film, whereby operation failure tends to be caused, it is preferable that the internal section except the outermost surface at the outer portion of the endoscope is made of a water-impermeable polymer.

It is a feature in the capsule endoscope of the present invention that the surface area has a wettability index of at least 40 mN/m.

The wettability index in the present invention is obtained by a test method of evaluating a hydrophilicity degree. Numeric value (mN/m) of surface tension of a mixed solvent which is determined to wet the polymer member surface after coating a bunch of mixed solvents (formamide and ethylene glycol monoethylether, for example) having different values of surface tension in order on the surface of the polymer member is defined as a wettability index. In accordance with a specified method in JIS K 6768 (The wettability test method of polyethylene and polypropylene), a cotton-tipped swab is soaked with a commercially available wettability standard reagent (31 - 54 mN/m; the larger the value is, the more improved hydrophilicity) to conduct measurement after coating the resulting on the surface of a test specimen.

A polymer constituting the surface area having a wettability index of at least 40 mN/m in the present invention is not specifically limited, but a polymer having a nonionic group or an anionic group is preferable. Further, as a nonionic group, at least one of a sugar chain structure having a single hydroxyl group or a plurality of hydroxyl groups, and alkylene oxide is preferable. As an anionic group, a sulfonic acid group or a phosphoric acid group is preferable.

Examples of such the polymer include acrylic acid alkyl esters such as methyl methacrylate and so forth; unsaturated olefin polymers such as ethylene, propylene, butadiene, isoprene and so forth; cycloolefin polymers copolymrized with the unsaturated olefin; a homopolymer of the cycloolefin polymer such as a norbornene resin; polycarbonates, styrene, a vinyl acetate polymer, a phenol resin, a silicon resin, and so forth.

The surface on which a hydrocarbon group on the polymer surface is substituted by a hydroxyl group can be obtained by treating the polymer of the present invention in the presence of oxygen. After modifying the surface so as to possess a hydroxyl group, it can be modified so as to possess an alkylene oxide group via reaction of alkylene oxide or a derivative thereof. A substituent having a sulfonic acid group can be introduced employing a hydroxyl group to conduct a hydrophilic treatment. This surface modification can produce a desired result in the depth range of 1 - 1000 nm from the surface.

Since any of the above-described polymers of the present invention exhibits low hydrophilicity, the surface is preferably subjected to a hydrophilic treatment. Examples of the hydrophilic treatment include a corona discharge treatment, a plasma discharge treatment, an arc discharge treatment, a flame treatment and so forth, but a corona discharge treatment and a plasma discharge treatment are preferable in view of the resulting hydrophilic effect and ease of hydrophilicity-providing.

The corona discharge treatment is referred to as a treatment conducted by applying a high voltage of at least 1 kV between electrodes at atmospheric pressure to discharge, and commercially available apparatuses produced by Kasuga electric works Ltd., and Toyo electric Corporation are used to conduct the corona discharge treatment. A frequency of 20 - 100 kHz is used for the corona discharge treatment, and a frequency of 30 - 60 kHz is preferably used. When frequency drops, the corona discharge treatment can not be evenly conducted, whereby unevenness in the corona discharge treatment is generated. On the other hand, when frequency becomes higher, no specific problem is caused in the case of conducting a high power corona discharge treatment, but it becomes difficult to conduct the treatment stably in the case of conducting a low power corona discharge treatment, and as a result, unevenness in the corona discharge treatment is also generated.

The plasma discharge treatment can be conducted by exposing a polymer substrate in the high frequency discharge atmosphere under argon or oxygen atmosphere employing a plasma discharge treatment apparatus. An atmospheric pressure plasma discharge treatment method of applying a high frequency pulse voltage, which is described in Japanese Patent O.P.I. Publication No. 11-181573, Japanese Patent O.P.I. Publication No. 2000-26632 and Japanese Patent O.P.I. Publication No. 2002-110397, can be used as an atmospheric pressure plasma discharge treatment method.

As an example of an application method for a capsule endoscope in the present invention, the endoscope inspection for early detection of cancer at the upper and lower portions in the inside of digestive organs with the above-described endoscope observation system will be described. A doctor initially prepares an endoscope observation system, and then asks an object undergoing an inspection to fast 8 hours to swallow a tablet as capsule endoscope 1. Subsequently, it is given to the inside of an oral cavity with a small amount of water, and moved smoothly in the inside of digestive organs to start wireless transmission of images, and also to start storing image data. In some cases, these data are displayed on the image-plane of a liquid crystal monitor or a home TV.

### EXAMPLE

Next, the present invention will be explained referring to examples, but the present invention is not limited thereto. Incidentally, in the examples, "parts" and "%" are "parts by weight" and "% by weight", respectively, unless otherwise specifically mentioned.

### Example 1

### «Preparation of capsule endoscope»

### [Preparation of capsule endoscope 101]

Prepared was capsule endoscope 101 composed of a structure integrated with an objective lens, an illuminating source section, a solid-state image sensor and so forth described in Fig. 1, and installed via mold of a thickness of 200 µm, in which protective member S was crosslinked with phenyldiisocyanate, and partly-hydrophobicity-treated water-resistant-treatment gelatin was employed. The surface of the protective member formed from this partly-hydrophobicity-treated gelatin had a wettability index of 38 mN/m.

### [Preparation of capsule endoscope 102]

Prepared was capsule endoscope 102 composed of a structure integrated with an objective lens, an illuminating source section, a solid-state image sensor and so forth described in Fig. 1, and installed via mold of a thickness of 200 µm, in which methyl polymethacrylate (a resin obtained by polymerizing a monomer produced by Mitsubishi Gas Chemical Co., Inc.: PMMA) as protective member S was employed. The surface of the protective member formed from this methyl polymethacrylate had a wettability index of 32 mN/m.

### [Preparation of capsule endoscope 103]

Prepared was capsule endoscope 103 composed of a structure integrated with an objective lens, an illuminating source section, a solid-state image sensor and so forth described in Fig. 1, and installed via mold of a thickness of 200 µm, in which a norbornene resin (ZEONOR 1020R produced by Zeon Corporation: COP) as protective member S was employed. The surface of the protective member formed from this norbornene resin had a wettability index of 32 mN/m.

### [Preparation of capsule endoscopes 104 - 106]

Capsule endoscopes 104 - 106 were prepared similarly to preparation of capsule endoscopes 101 - 103, except that in preparation capsule endoscopes 101 - 103, the area corresponding to 50% of the total area of protective member S was corona-discharge-treated at an output of 12 kv for one minute employing a corona discharge treatment apparatus (manufactured by Kasuga electric works Ltd.), and a wettability index of the surface was set to 54 mN/m.

### [Preparation of capsule endoscope 107]

Capsule endoscope 107 was prepared similarly to preparation of capsule endoscope 104, except that in preparation capsule endoscope 104, water-resistant-treatment gelatin obtained by crosslinking protective member S with phenyldiisocyanate was replaced by a polypropylene resin (PP).

### [Preparation of capsule endoscope 108]

Capsule endoscope 107 was prepared similarly to preparation of capsule endoscope 104, except that in preparation capsule endoscope 104, water-resistant-treatment gelatin obtained by crosslinking protective member S with phenyldiisocyanate was replaced by a polyethylene resin (PP).

### [Preparation of capsule endoscopes 109 - 113]

Capsule endoscopes 109 - 113 were prepared similarly to preparation of capsule endoscopes 104 - 108, except that in preparation capsule endoscopes 104 - 108, the area corresponding to 50% of the total area of protective member S was plasma-treated so as to give the surface having a wettability index of 54 mN/m employing a plasma discharge treatment apparatus [power: 500 W, generation source: microwave having a frequency of 2.45 GHz, treatment pressure: 40 Pa (0.3 Torr)] manufactured by Shibaura Mechatronics Corporation in place of a corona discharge treatment apparatus.

### [Preparation of capsule endoscopes 114 - 119]

Capsule endoscopes 114 - 119 were prepared similarly to preparation of capsule endoscope 110, except that in preparation of capsule endoscope 110 (base polymer: PMMA), the treatment area subjected to the following E treatment after conducting the above-described plasma discharge treatment was replaced by one described in Table 1.

E treatment means that the PMMA surface is alkali-hydrolyzed with sodium hydroxide liquid of pH12 subsequently to conduct polyoxyethylenation (6 repeating units) with ethylene oxide.

### [Preparation of capsule endoscopes 120 - 122]

Capsule endoscopes 120 - 122 were prepared similarly to preparation of above-described capsule endoscope 119, except that in preparation of capsule endoscope 119 (treatment area: 100%), E treatment was replaced by the following T treatment, S treatment or P treatment.

T treatment means that after the PMMA surface is alkali-hydrolyzed with sodium hydroxide liquid of pH12, hydroxyethyl cellulose is dissolved in a methylethyl ketone solvent, and the resulting was coated 1.0 µm thick and dried to produce a sugar chain structure.

S treatment means that the PMMA surface is alkali-hydrolyzed with sodium hydroxide liquid of pH12 subsequently to conduct a sulfonation treatment with a sulfonic acid.

P treatment means that the PMMA surface is alkali-hydrolyzed with sodium hydroxide liquid of pH12 subsequently to conduct a phosphorylation treatment with a phosphoric acid.

### [Preparation of capsule endoscope 123]

Capsule endoscope 123 was prepared similarly to preparation of above-described capsule endoscope 107, except that in preparation of capsule endoscope 107 (base polymer: PP), E treatment (treatment area: 100%) was conducted after conducting a corona discharge treatment.

### «Evaluation of capsule endoscope»

### [Measurement of wettability index of surface]

The wettability index of the surface of a protective member in each capsule endoscope prepared above was measured in accordance with the following method.

In accordance with a specified method in JIS K 6768 (The wettability test method of polyethylene and polypropylene), a cotton-tipped swab is soaked with a commercially available wettability standard reagent (31 - 54 mN/m; the larger the value is, the more improved hydrophilicity) to measure a value of 0.5 seconds after coating the resulting on the surface of a test specimen, and maintaining the surface temperature at 37 °C. In this case, the minimum value of reagent No. sufficiently wetted with no water repellency was designated as the wettability index.

### [Evaluation of durability]

After cow's gastric fluid collected in advance which has been kept in a freezer is thawed, and each capsule endoscope prepared above is immersed in this gastric fluid for one hour, transmission/reception of a photographed image is conducted to evaluate whether or not failure has been produced. Criteria are as follows. Good: in the case of transmission with no failure, and N/G: in the case of transmission failure.

### [Evaluation of sharpness of photographed image]

Each capsule endoscope prepared above was moved in the inside of a simulant body from a gastric region to small intestine in a human body, photographed images were transmitted during movement, and sharpness of the received photographed image was visually observed to evaluate sharpness of the photographed image according to the following criteria.

5: The transmitted photographed image is an excellent sharp image, accompanied with no foreign material adhesion to a protective member.

4: The transmitted photographed image is a sharp image, accompanied with no foreign material adhesion to a protective member.

3: The transmitted photographed image is an image in which degradation of sharpness is slightly observed because of foreign material adhesion partly to a protective member, but is a practically workable image.

2: The transmitted photographed image is an image in which degradation of sharpness is observed because of foreign material adhesion to a protective member, and the image level is below a practically tolerable image level.

1: The transmitted photographed image is an image in which degradation of sharpness is largely observed because of a great deal of foreign material adhesion to a protective member, and the image level is practically intolerable.

**Table 1**

| Capsule endoscope No. | Structure of protective member | | | Evaluation results | | | Remarks |
|---|---|---|---|---|---|---|---|
| | Base polymer | Surface treatment | Treatment area ratio (%) | Wettability index (mN/m) | *1 | *2 | |
| 101 | Gelatin | None | - | 38 | N/G | 1 | Comp. |
| 102 | PMMA | None | - | 32 | Good | 1 | Comp. |
| 103 | COP | None | - | 32 | Good | 1 | Comp. |
| 104 | Gelatin | (C) | 50% | 54 | Good | 3 | Inv. |
| 105 | PMMA | (C) | 50% | 54 | Good | 3 | Inv. |
| 106 | COP | (C) | 50% | 54 | Good | 3 | Inv. |
| 107 | PP | (C) | 50% | 54 | Good | 3 | Inv. |
| 108 | PE | (C) | 50% | 54 | Good | 3 | Inv. |
| 109 | Gelatin | (P) | 50% | 54 | Good | 3 | Inv. |
| 110 | PMMA | (P) | 500 | 54 | Good | 3 | Inv. |
| 111 | COP | (P) | 50% | 54 | Good | 3 | Inv. |
| 112 | PP | (P) | 50% | 54 | Good | 3 | Inv. |
| 113 | PE | (P) | 50% | 54 | Good | 3 | Inv. |
| 114 | PMMA | (P) + E treatment | 50% | 54 | Good | 3 | Inv. |
| 115 | PMMA | (P) + E treatment | 20% | 54 | Good | 3 | Inv. |
| 116 | PMMA | (P) + E treatment | 40% | 54 | Good | 3 | Inv. |
| 117 | PMMA | (P) + E treatment | 60% | 54 | Good | 3 | Inv. |
| 118 | PMMA | (P) + E treatment | 80% | 54 | Good | 4 | Inv. |
| 119 | PMMA | (P) + E treatment | 100% | 54 | Good | 5 | Inv. |
| 120 | PMMA | (P) + T treatment | 100% | 54 | Good | 5 | Inv. |
| 121 | PMMA | (P) + S treatment | 100% | 54 | Good | 5 | Inv. |
| 122 | PMMA | (P) + P treatment | 100% | 54 | Good | 5 | Inv. |
| 123 | PP | (C) + E treatment | 100% | 54 | Good | 5 | Inv. |

| | | | | | | | |
|---|---|---|---|---|---|---|---|
| *1: Durability, *2: Sharpness of photographed image, (P): Plasma discharge treatment, (C): Corona discharge treatment, Comp.: Comparative example, Inv.: Present invention | | | | | | | |

As is clear from results described in Table 1, it is to be understood that capsule endoscopes of the present invention including the surface area having a wettability index of at least 40 mN/m exhibit excellent durability in the situation of storage for a long duration under the condition of strong acidity, and also exhibit excellent resistance of adhesion of a material in the inside of body cavity to a protective member at a photographed site to transmit extremely sharp images.

### Example 2

Capsule endoscopes 204 - 223 were prepared similarly to preparation of capsule endoscopes 104 - 123, except that for the structure of an observation optical system and an illumination optical system, a light source section cover (made of a norbornene resin) and an objective lens (made of a norbornene resin) were placed on the outermost surface as shown in Fig. 2, the surfaces of the light source section cover and the objective lens were surface-treated similarly to preparation of capsule endoscopes 104 - 123 in Example 1. Further, durability and photographed image sharpness were evaluated in the same method as described in Example 1, and as a result, the same excellent results as in Example 1 were able to be obtained.

## Claims

1. A capsule endoscope comprising surface area A having a wettability index of at least 40 mN/m.

2. The capsule endoscope of Claim 1,
wherein the surface area A is formed from a protective member of a photographing section comprising a lens and an illumination unit.

3. The capsule endoscope of Claim 1,
wherein the surface area A is a lens surface or an illumination unit surface.

4. The capsule endoscope of any one of Claims 1 - 3,
wherein the surface area A is corona-discharge-treated or plasma-discharge-treated.

5. The capsule endoscope of any one of Claims 1 - 4,
wherein a material constituting the surface area A is a polymer having a nonionic group or an anionic group.

6. The capsule endoscope of Claim 5,
wherein the nonionic group comprises a sugar chain structure having a single hydroxyl group or a plurality of hydroxyl groups, or an alkylene oxide group.

7. The capsule endoscope of Claim 5,
wherein the anionic group is a sulfonic acid group or a phosphoric acid group.
